Europäisches Patentamt

European Patent Office

Office européen des brevets

(19)

(11) Publication number: **0 089 823**
**B1**

(12) **EUROPEAN PATENT SPECIFICATION**

(45) Date of publication of patent specification: **17.12.86**

(51) Int. Cl.⁴: **G 01 M 3/08,** A 61 B 1/00,
**G 01 M 3/26**

(21) Application number: **83301508.4**

(22) Date of filing: **18.03.83**

(54) **Leakage detector of endoscopes.**

(30) Priority: **19.03.82 JP 44273/82**

(43) Date of publication of application:
**28.09.83 Bulletin 83/39**

(45) Publication of the grant of the patent:
**17.12.86 Bulletin 86/51**

(84) Designated Contracting States:
**AT BE CH DE FR GB IT LI NL SE**

(56) References cited:
**EP-A-0 028 396**
**WO-A-81/01332**

(73) Proprietor: **OLYMPUS OPTICAL CO., LTD.**
**43-2, 2-chome, Hatagaya Shibuya-ku**
**Tokyo 151 (JP)**

(72) Inventor: **Shimizu, Yoshihito**
**c/o Nishihachi-Corporas 241 5-16-2, Sanda-Cho**
**Hachioji-Shi Tokyo (JP)**

(74) Representative: **Jones, Colin et al**
**W.P. THOMPSON & CO. Coopers Building**
**Church Street**
**Liverpool L1 3AB (GB)**

Courier Press, Leamington Spa, England.

## Description

The present invention relates to an endoscope system, and more particularly, to a leakage detector for endoscopes of the watertight type.

For sanitary reasons, endoscopes are invariably washed and sterilized after use. Taking into consideration the convenience of washing and the like, there have been provided endoscopes of an entirely watertight structure including operating devices thereof so that it is possible easily to wash and sterilize the whole endoscope body by immersing it in washing liquid and an antiseptic solution. In this case, however, there is no problem when the endoscope is of an intact watertight structure but, when there are defects in the watertight structure of the operating devices or pinholes and cracks in a sheath tube or the like of an endoscope flexible tube, the washing liquid leaks into the inside of the endoscope. Under this situation, corrosion is caused from the inside of the endoscope and the washing liquid can penetrate into a bundle of optical fibres, resulting in hindrance to transmission of light. In addition, there is a possibility of having an adverse effect upon patients due to outward leakage of washing liquid which has previously leaked into the inside of the endoscope. To prevent such an effect, as disclosed in US—A—4216767 the internal pressure in the endoscope may be raised to greater than the external pressure by providing an opening for applying pressure to the endoscope and by applying compressed air to such opening, whereby the leakage of liquid into the endoscope when immersed in a washing liquid is prevented. According to this method, it is advantageously possible to find easily any location where air comes out in the form of bubbles from pinholes, if any.

However, it is necessary properly to choose a value of pressure to be used when compressed air is applied, otherwise there would be a likelihood that the endoscope itself may be damaged by the pressure. To control the pressure using a pump, the apparatus becomes disadvantageously complex.

In addition, it is extremely inconvenient that, in order to apply compressed air to the endoscope, a pump for use exclusively with the endoscope is always provided.

EP—A2—0028396 describes an endoscope of the airtight type in which the endoscope connector, adapted to be coupled to the scope socket of a light source apparatus has a cylindrical portion and an air port which communicates with the interior of the endoscope. Before immersing the endoscope in a washing or disinfecting solution, a fluid-proof cap is slipped over the cylindrical portion of the endoscope connector and is fastened in place. The cap is sealed to such cylindrical portion so that it acts like a pump to place the endoscope interior under super atmospheric pressure. The washing or disinfecting solution is thereby prevented from reaching the interior of the endoscope and any leakage is revealed by bubbles coming from the leakage point. However the reliability of such a leakage detecting means is open to question as there is no simple way of checking the effectiveness of the seal between the cap and the cylindrical portion of the endoscope connector and, should the air pressure in the endoscope be dissipated rapidly, not only would a leak be not revealed but the washing or disinfecting solution could reach the interior of the endoscope.

It is an object of the present invention to provide a simply constructed leakage detecting means for endoscopes of the watertight type, whereby the endoscope is subjected to internal pressure when submerged in a liquid for performing a leakage test.

The present invention resides in a endoscope system comprising an endoscope of the watertight type having a compressed air inlet portion, a light source apparatus having a scope socket to which a connector of the endoscope may be coupled for conducting light into the endoscope, and means for detecting a leakage point in the endoscope by applying compressed air to the endoscope and immersing it in a tank of liquid so that any leakage is revealed by the appearance of bubbles, and is characterised in that the leakage detecting means comprises a first connector member constructed to be connected to the scope socket; a second connector member adapted to be coupled to said compressed air inlet portion for feeding compressed air to the interior of the endoscope when carrying out a leakage test; a communication tube interconnecting said first and second connector members; and a pressure regulator which is provided in one of said first and second connector members or in said connecting tube, the light source apparatus being provided with an air supply apparatus for feeding air to the endoscope via the scope socket when coupled to the endoscope connector, such that, when carrying out a leakage test with the scope socket coupled to the first connector member, the air supply apparatus of the light source apparatus supplies to the interior of the endoscope compressed air whose pressure is regulated by the pressure regulator.

Thus, damage to the endoscope due to the application of too high a pressure is avoided.

A pressure regulator screw may be used as a retainer for a valve spring of a relief valve which serves as the pressure regulator. Thus, it is possible to adjust the applied pressure depending upon different types of endoscopes, thus increasing universality of the leakage detector.

The invention is further described, by way of example, with reference to the accompanying drawings, in which:—

Fig. 1 is a perspective view illustrating use of a leakage detector according to the invention;

Fig. 2 is a sectional view of a leakage detector according to one embodiment of the invention;

Fig. 3 is a sectional view of a leakage detector according to another embodiment of the invention; and

Fig. 4 is a sectional view of a leakage detector according to a further embodiment of the invention.

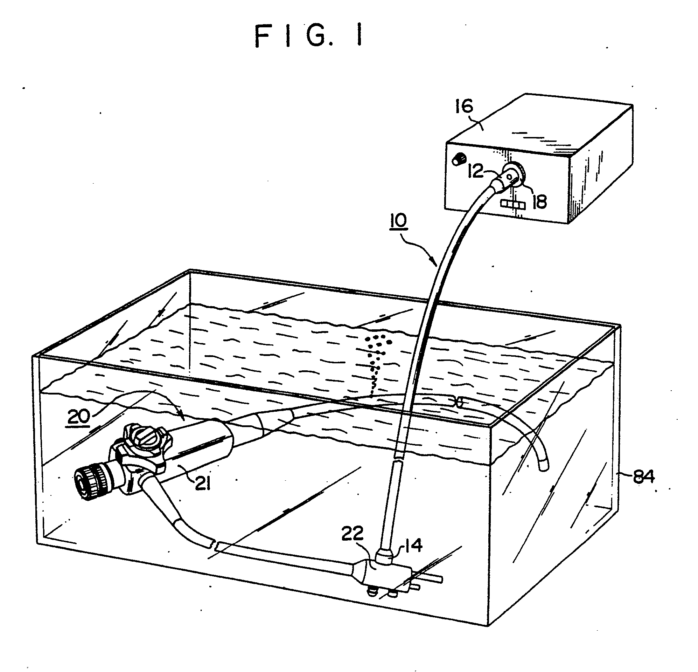

Referring now to Fig. 1, a leakage detecting means or leakage detector 10 is formed with a connector, one end of which has a first connector member 12 and the other end of which has a second connector member 14. The first connector member 12 is connected to a scope socket 18 of a light source apparatus 16 for an endoscope which includes an air supply apparatus. The second connector member 14 is connected to a connector 22 of an endoscope 20 to be tested for leaks by immersion in a tank 84 of liquid with the endoscope under internal pressure.

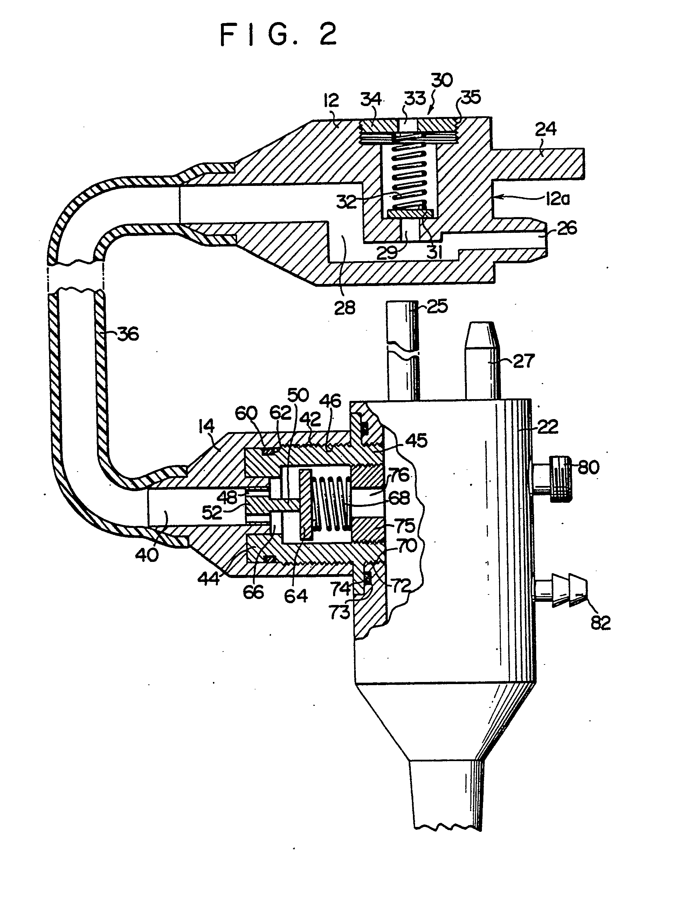

Referring now to Fig. 2, which illustrates the internal structure of the leakage detector 10 of the first embodiment of the invention, which is connected as shown in Fig. 1, the first connector member 12 thereof has substantially the same shape and positional relationship as the connector 22 of the endoscope. One end surface 12a of the first connector member 12 is provided with a dummy inlet piece 24, equivalent in external shape to a light guide piece 25 of the endoscope, and a port 26 for admitting compressed air which has the same shape as a piece 27 for admitting air into an air passage (not shown) of the endoscope. The dummy inlet piece 24 serves as a guide pin when the first connected member 12 is fixed to the light source apparatus 16. The port 26 through which compressed air is admitted from an air supply apparatus (not shown) in the light source apparatus 16, communicates with a first passage 28 which conducts compressed air.

The inside of the first connector member 12 is provided with a relief valve member 30 communicating with the first passage 28 for maintaining the applied air pressure from the light source apparatus 16 constant. The relief valve member 30 is composed of a relief valve element 31 for closing a relief port 29 branching from a first passage 28, a coiled spring 32 which presses the valve element 31 and a laminar spring retainer 34 having a hole 33. The spring retainer 34 is provided with male threads 35 on its periphery so that the force of the spring 32 can be adjusted in accordance with the depth to which the retainer 34 is screwed into the connector member 12.

The first passage 28 is hermetically connected to one end of a tube 36, which forms an air admission passage and communicates with a second passage 40 which is provided in the second connector member 14 by hermetically connecting the other end of tube 36 to the second passage 40. The second connector member 14 is provided with female threads 42 on its end inside thereof so as to be threaded on male threads 46 provided on the outside periphery of a compressed air admitting portion 44 which projects from the connector 22 of the endoscope. Additionally, the inside of the second connector member 14 is provided with a compressed air applying portion 52 which enters a central port 66 (to be described later) and which is provided with a projection 50 having a plurality of communicating holes 48 about its circumference.

The compressed air admitting portion 44 of the endoscope has the inlet port 66 at its central axis and the inlet port is closed by means of an inlet valve element 64 which is biased by a coiled spring 68. A spring retainer 75 which supports the spring 68 is threaded into the admitting portion 44 and is provided with a through-hole 76 communicating the inlet port 66 with the inside of the endoscope. A sealing ring 60 is disposed in a circumferential groove 62 provided on the outside periphery of the admitting portion 44 so as to keep the joint airtight. The admitting portion 44 is integrally attached to the connector 22 by a male thread 70 which is provided on the outside periphery of a fitting portion 45 and a female thread 72 which is provided on the inside periphery of through-hole of the connector 22. In order to maintain the airtightness of the attached portion between the connector 22 and the portion 44, a sealing ring 74 is disposed in a circumferential groove 73 provided around the through-hole 76 of the connector 22.

In addition, an inlet piece 80 for admitting air and water and an outlet piece 82 for suction are provided on the side of the connector 22.

The leakage detector endoscopes constructed as described above and as shown in Fig. 1, is used to detect pinholes or the like by connecting the first connector member 12 to the light source apparatus 16 for endoscopes, connecting the second connector member 14 to the compressed air admitting portion 44 of connector 22 of the watertight endoscope 20 and then immersing the endoscope 20 into a tank 84 holding water (or cleaning liquid, antiseptic solution).

In operation, the first connector member 12 is connected to the scope socket 18 which forms an air supply portion of the light source apparatus 16, so that the leakage detector uses compressed air normally to be supplied when the air admission piece 27 of the connector 22 is connected to the scope socket 18 with air and water being supplied during use of the endoscope. The compressed air applied from the light source apparatus 16 is led through the port 26 of the first connector member 12, the first passage 28 and the tube 36 to the second passage 40 within the second connector member 14. When the second connector member 14 is threaded into the air admission portion 44 of the connector 22, the inlet valve element 64 within the portion 44 is pushed in by means of the projection 50 within the second connector member 14 so that the inside of the portion 44 communicates with the second passage 40 through the holes 48 of the air applying portion 52. As a result, compressed air from the light source apparatus 16 is supplied through the second passage 40, the inside of the portion 44 and the through-hole 76 to the inside of the connector 22. Consequently, the compressed air from the light source apparatus 16 is applied to the inside of the endoscope 20 so that it is possible to detect locations at a glance where defects, such as pinholes, if any, exist on the outside surface thereof, since bubbles are produced from the defects.

When there is no defect in the endoscope so that airtightness is maintained the relief valve element 31 for maintaining the air pressure applied to the endoscope constant is opened since the spring 32 yields to the applied air pressure when it exceeds a given level. As a result, it is possible to maintain the applied pressure constant, to prevent the endoscope from being damaged and to inspect always with a constant applied pressure. In addition, the spring retainer 34 makes it possible for the initial force of spring 32 to be adjusted depending upon the endoscopes to be tested.

During the leakage detection, washing liquid enters the air admission piece 27 and thereby reaches the inside of the air passage (not shown) of the endoscope for the perfect watertight endoscope 20, the inlet piece 80 for air and water and the outlet suction piece 82. However, since these parts form a passage system communicating the inlet port with the outlet port of the tip end of the endoscope, washing liquid does not penetrate into the interior proper of the endoscope but it is preferred rather that washing liquid enters from the inlet port to the passage system since the inside of the endoscope is washed simultaneously with the outside.

No washing liquid penetrates into the light guide piece 25 of the connector 22 since a waterproofing is applied thereto.

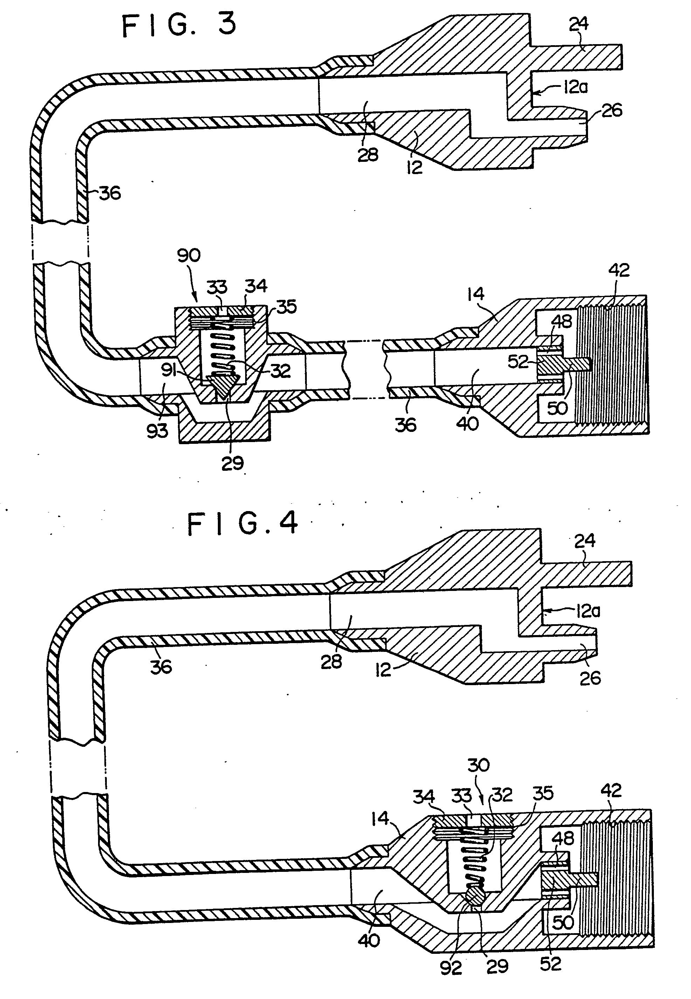

A second embodiment of the leakage detector according to the invention will now be described by referring to Fig. 3. Like reference characters designate corresponding parts of the first embodiment and the description of such corresponding parts with reference to Fig. 3 will be omitted.

In the second embodiment, a relief valve portion is not provided within the first connector member 12 but a relief valve portion 90 is provided in the middle of the tube 36, within which a conical-shaped relief valve element 91 is provided. The relief valve portion 90 has a passage 93 in the side thereof, both ends of which are open and are connected to tubes 36. The other structures are similar to those of the first embodiment. A relief port 29 branching off from the passage 93 is closed by a valve element 91 which is biassed by a spring 32. The spring force is adjusted by an adjusting screw 35 of a spring retainer 34. According to this embodiment, advantages are that, since a relief valve is not provided within connector members, the connector members can be made of a reduced size and, when the relief valve is located within water, the action of the relief valve can be recognised through bubbles produced. The action of the relief valve element 91, similar to that in the first embodiment, is to permit air to be vented when the air pressure within the endoscope 20 exceeds a given value, so avoiding possible damage to the endoscope.

Referring now to Fig. 4, a third embodiment of the invention will be described. As like reference characters designate corresponding parts of the first embodiment, the description of such corresponding parts with reference to Fig. 4 will be omitted. In this embodiment, a relief valve portion is provided within the second connector member 14 and a relief port 29 branches off from a second passage 40. The relief port 29 is closed by a ball-shaped relief valve element 92 which is biassed by a spring 32, similar to the first embodiment. The spring force is adjustable by means of an adjusting screw 35.

According to this embodiment, since the relief valve is provided within the second connector member 14, it is advantageously possible to certainly recognize the action of the relief valve element 92 through the appearance of bubbles. The action of the relief valve element 92 is the same as that of the relief valve element 31 in the first embodiment.

## Claims

1. An endoscope system comprising an endoscope (20) of the watertight type having a compressed air inlet portion (44), a light source apparatus (16) having a scope socket (18) to which a connector (22) of the endoscope may be coupled for conducting light into the endoscope (20), and means (10) for detecting a leakage point in the endoscope (20) by applying compressed air to the endoscope (20) and immersing it in a tank (84) of liquid so that any leak is revealed by the appearance of bubbles, characterised in that the leakage detecting means (10) comprises a first connector member (12) constructed to be connected to the scope socket (18); a second connector member (14) adapted to be coupled to said compressed air inlet portion (44) for feeding compressed air to the interior of the endoscope when carrying out a leakage test; a communication tube (36) interconnecting said first and second connector members (12, 14); and a pressure regulator (30, 90) which is provided in one of said first and second connector members (12, 14) or in said communicating tube (36), the light source apparatus (16) being provided with air supply apparatus for feeding air to the endoscope (20) via the scope socket (18) when coupled to the endoscope connector (22), such that, when carrying out a leakage test with the scope socket (18) coupled to the first connector member (12), the air supply apparatus of the light source apparatus (16) supplies to the interior of the endoscope (20) compressed air whose pressure is regulated by the pressure regulator (30, 90).

2. An endoscope system according to claim 1, in which said second connector member (14) has a compressed air supplying portion (52), which has a projection (50) arranged to open an inlet valve (64) which is adapted to close said inlet portion (44) of the endoscope (10) when the second connector member (14) is mounted on said compressed air inlet portion (44), so as to allow the inside of said inlet portion (44) to communicate with said communicating tube (36).

3. An endoscope system according to claim 1 or 2, in which said pressure regulator comprises a relief valve (30, 90) for automatically regulating the compressed air pressure within the endoscope to a given value which is sufficient to damage the endoscope (20) when applied to its interior.

## Patentansprüche

1. Endoskop-System, umfassend ein Endoskop (20) wasserdichter Bauart mit einem Druckluft-Einlaßabschnitt (44), einen Lichtquellenapparat (16) mit einer Sichtgerät-Buchse (18), an die ein Stecker (22) des Endoskops zum Einleiten von Licht in das Endoskop (20) anschließbar ist, und eine Einrichtung (10) zum Erfassen einer Leckstelle in dem Endoskop (20) durch Beaufschlagung des Endoskops (20) mit Druckluft und Eintauchen desselben in einen Flüssigkeitstank (84), so daß ein etwaiges Leck durch das Auftreten von Blasen offenbar wird, dadurch gekennzeichnet, daß die Leckerfassungseinrichtung (10) ein erstes Steckerglied (12) umfaßt, das so aufgebaut ist, daß es sich an die Sichtgerät-Buchse (18) anschließen läßt; ein zweites Steckerglied (14), das so ausgelegt ist, daß es an den Druckluft-Einlaßabschnitt (44) zur Zuführung von Druckluft in das Innere des Endoskops bei der Durchführung eines Lecktests anschließbar ist; ein das erste und das zweite Steckerglied (12, 14) miteinander verbindendes Verbindungsrohr (36); und einen in dem ersten oder dem zweiten Steckerglied (12, 14) oder in dem Verbindungsrohr (36) vorgesehenen Druckregler (30, 90), wobei der Lichtquellenapparat (16) mit einem Luftzuführapparat versehen ist, um dem Endoskop (20) über die Sichtgerät-Buchse (18) Luft zuzuführen, wenn diese mit dem Endoskop-Stecker (22) gekoppelt ist, so daß bei Durchführung eines Lecktests und mit dem ersten Steckerglied (12) gekuppelter Sichtgerät-Buchse (18) der Luftzuführapparat des Lichtquellenapparats (16) in das Innere des Endoskops (20) Druckluft einleitet, deren Druck durch den Druckregler (30, 90) geregelt ist.

2. Endoskop-System nach Anspruch 1, wobei das zweite Steckerglied (14) einen Druckluft-Zuführabschnitt (52) mit einem Vorsprung (50) aufweist, der so angeordnet ist, daß er ein zum Schließen des Einlaßabschnitts (44) des Endoskops (10) ausgelegtes Einlaßventil (64) öffnet, wenn das zweite Steckerglied (14) an dem Druckluft-Enlaßabschnitt (44) montiert wird, damit das Innere des Einlaßabschnitts (44) mit dem Verbindungsrohr (36) in Verbindung treten kann.

3. Endoskop-system nach Anspruch 1 oder 2, wobei der Druckregler ein Entlastungsventil (30, 90) umfaßt, um den Luftdruck innerhalb des Endoskops automatisch auf einen gegebenen Wert zu regeln, der zu gering ist, um das Endoskop (20) bei Beaufschlagung von dessen Innern zu beschädigen.

## Revendications

1. Dispositif d'endoscopie comprenant un endoscope (20) du type étanche à l'eau et muni d'une partie d'entrée d'air comprimé (44), un appareil formant source lumineuse (16) qui possède une douille d'endoscope (18) à laquelle un connecteur (22) de l'endoscope peut être accouplé pour introduire la lumière dans l'endoscope (20), et des moyens (10) permettant de détecter un point de fuite dans l'endoscope (20), en introduisant de l'air comprimé dans l'endoscope (20) et en l'immergeant dans un bac de liquide (84), de sorte qu'une fuite éventuelle est détectée par l'apparition de bulles, caractérisé par le fait que les moyens de détection des fuites (10) comprennent un premier élément de connecteur (12) agencé pour être connecté à la douille (18) de l'endoscope; un deuxième élément de connecteur (14) agencé pour être accouplé à la dite partie d'entrée d'air comprimé (44), pour introduire l'air comprimé dans l'espace intérieur de l'endoscope lorsqu'on exécute un contrôle de fuite; un tube de liaison (36) qui interconnecte le premier et le deuxième éléments de connecteurs (12, 14); et un régulateur de pression (30, 90) qui est prévu dans l'un des premier et deuxième éléments de connecteurs (12, 14) ou dans ledit tube de liaison (36), l'appareil formant source lumineuse (16) étant équipé d'un appareil d'alimentation en air destiné à introduire de l'air dans l'endoscope (20) par l'intermédiaire de la douille d'endoscope (18) lorsque cette dernière est couplée au connecteur (22) de l'endoscope de telle sorte que, lorsqu'on effectue un contrôle de fuite avec la douille d'endoscope (18) couplée au premier élément de connecteur (12), le dispositif d'alimentation en air du dispositif source lumineuse (16) envoie dans l'espace intérieur de l'endoscope de l'air comprimé dont la pression est réglée par un régulateur de pression (30, 90).

2. Dispositif d'endoscopie selon la revendication 1, caractérisé par le fait que ledit deuxième élément de connecteur (14) comprend une partie (52) d'alimentation en air comprimé qui possède une saillie (50) agencée pour ouvrir une soupape d'entrée (64) qui est agencée pour fermer ladite partie d'entrée (44) de l'endoscope (20) lorsque le deuxième élément de connecteur (14) est monté sur ladite partie d'entrée d'air comprimé (44) de manière à permettre à l'espace intérieur de ladite partie d'entrée (44) de communiquer avec ledit tube de liaison (36).

3. Dispositif d'endoscopie selon l'une des revendications 1 et 2, caractérisé par le fait que ledit régulateur de pression comprend une soupape de sûreté (30, 90) destinée à régler automatiquement la pression d'air comprimé introduite dans l'endoscope à une valeur donnée qui est insuffisante pour endommager l'endoscope (20) lorsque cette pression est appliquée à l'espace intérieur de l'endoscope.

# F I G. 1

# F I G. 2

FIG. 3

FIG.4

3